# EUROPEAN PATENT SPECIFICATION

(11) **EP 1 065 193 B1**
(45) Date of publication and mention of the grant of the patent: **28.07.2004**
(21) Application number: 00202162.4
(22) Date of filing: 21.06.2000
(51) Int. Cl.: C07C 41/03, B01J 19/24, C08G 65/28

(54) **Method and device for manufacturing products in an industrial manner by means of an alcoxylation reaction**
Verfahren und Einrichtung zur industriellen Herstellung von Produkten durch Alkoxylationsreaktionen
Procédé et dispositif pour la fabrication industrielle de produits au moyen de réactions d'alkoxylation

(30) Priority: 02.07.1999 BE 9900457
(43) Date of publication of application: 03.01.2001
(73) Proprietor: INEOS, naamloze vennootschap, 2070 Zwijndrecht (BE)
(72) Inventor: Decadt, Ghislain, 1820 Steenokkerzeel (BE); Vercauteren, Carl, 9100 Sint-Niklaas (BE)
(74) Representative: Donné, Eddy

(56) References cited:
- DE-A- 4 037 284
- DE-A- 19 525 067
- FR-A- 1 054 451
- GB-A- 2 073 188

## Description

The present invention concerns a method and a device for manufacturing products in an industrial manner by means of an alcoxylation reaction, in particular an alkoxylation reaction whereby an ethoxylation and/or propoxylation takes place by making react alcohol on the one hand with ethylene oxide and/or propylene oxide on the other hand.

In particular, it concerns the industrial production of detergent alcohol ethoxylates, what are called surfactants, and polyethylene glycols which become liquid at reaction temperature.

It is known that, in order to produce alcohol ethoxylates, use can be made of a mixing vessel reactor with an external cooling circuit, whereby the liquid mass is pumped round over a cooler. An amount of alcohol is hereby provided in the mixing vessel, and ethylene oxide is subsequently added to it. The ethylene oxide can be supplied in the gas phase as well as in the liquid phase of the mixing vessel.

Instead of applying a mechanical mixing by means of a mixing gear, use can also be made of spray nozzles, whereby the product which is pumped round is atomised at the top of the reactor, so that a more intense contact is created with the gaseous ethylene oxide, which is supplied in the reactor at the top.

According to another known possibility, the ethylene oxide is mixed in the product which is pumped round by means of a venturi tube..

The above-mentioned techniques are all semi-batch processes, and they are disadvantageous in that the supply flow rate of the ethylene oxide is restricted. The reasons therefor can be found in the problems which arise due to the inflammability of the gaseous phase above the liquid in the reactor, as well as in the discharge of the considerable amount of exothermic reaction heat.

The above-mentioned known processes are also disadvantageous in that, because of the gaseous phase, the mass transport through the device is limited, and consequently also the general production speed. During the atomisation and mixing in the venturi tube, however, the contact between the gas and the liquid is more intense than in the mixing vessel reactor, but the mass transport is still the step which determines the speed.

The above-mentioned semi-batch processes are also disadvantageous in that large and expensive re-circulation coolers, as well as circulating pumps are required.

It is known that, as for example described in FR 1.054.451 and in GB 2.073.188, a number of the above-mentioned disadvantages can be remedied by making use of reactors in the shape of coil heat exchangers, whereby the reagents are made to flow through the tubes, whereas a continuous heat dissipation is provided for by making cooling liquid flow along the coil. The reactors are hereby operated in an isothermal manner. A disadvantage of this technique consists in that a perfect temperature regulation must constantly be provided for, and in that the used reactors are relatively expensive. Another disadvantage consists in that such reactors with coils cause a major pressure drop which counteracts the processing of the products in the reactor.

The invention aims a method and a device for manufacturing products in an industrial manner by means of an alkoxylation reaction, which are improved in relation to the known embodiment, in particular which are even more suitable for producing the products concerned by means of an industrial process. In particular, it aims a method and a device whereby the above-mentioned disadvantages of the known techniques are excluded.

To this aim, the invention in the first place concerns a method for manufacturing products such as surfactants and polyethylene glycols by means of an alkoxylation reaction, characterized in that it at least consists of putting the reagents together in the liquid phase, and of subsequently leading the reaction medium in which the reaction takes place at least for the greater part through one or several tube reactors, whereby in the tube reactor, the above-mentioned reaction is carried out in an adiabatic manner, and whereby a turbulence is created in the tube reactor by maintaining a Reynolds' number of at least 10⁵.

By putting the reagents together in the liquid phase and by subsequently letting the reaction take place in a tube reactor, a reaction can be realised at high flow rates, and consequently with a large mass discharge, as a result of which the restrictions of the known processes are excluded.

In practice, the invention is meant in particular for reactions whereby an ethoxylation and/or propoxylation takes place by making alcohol and/or another basic product on the one hand react with ethylene oxide and/or propylene oxide on the other hand.

It is also particularly useful in the case of alkoxylation reactions whereby there is a butoxylation.

The invention is particularly suitable among others for the production of alcohol alkoxylates of one or several of the following types:
a. primary alcohol ethoxylates 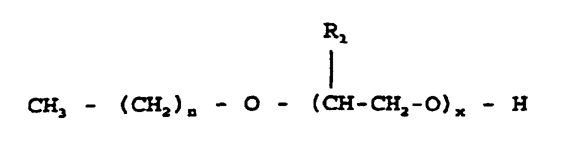
b. secondary alcohol ethoxylates 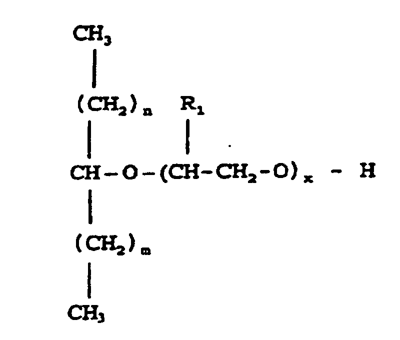
c. tertiary alcohol ethoxylates 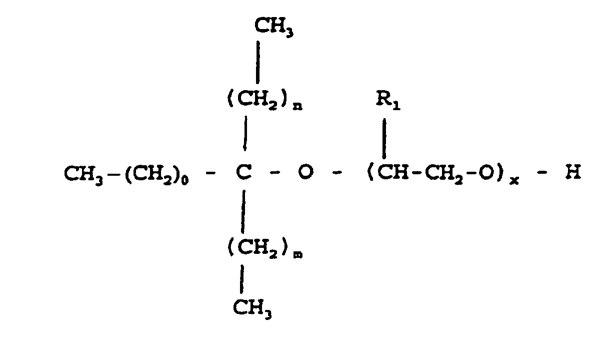 Whereby: R₁ consists of H, CH₃ or C₂H₅
   x = 0-50
   m,n,o = 0-20

The invention is also particularly suitable for the production of polyalkylene glycols, in particular of the following type: 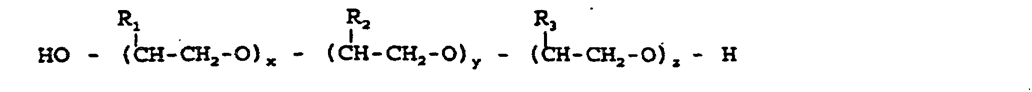 Whereby: R₁,R₂,R₃ consist of H, CH₃ or C₂H₅
x,y,z = 0-50

The method is preferably realised as a continuous process, although what is called a semi-batch process is not excluded.

As the reaction is carried out in an adiabatic manner, the disadvantages of the above-mentioned isothermal reaction, such as the strictly controlled cooling, are excluded. By making use of an adiabatic reaction, the process is accelerated thanks to the produced reaction heat, in other words, full advantage is taken of the increasing reaction rate at a rising temperature.

However, what precedes does not imply that coolings are excluded. Thus, for example, one or several coolings, for example intercoolings, can be applied so as to prevent the product temperature from rising above a certain value, which could possibly be disadvantageous to the quality of the end product.

By operating in an adiabatic manner in the reactors themselves, a number of operational problems which may occur during an isotherm process are excluded as well, such as for example the danger of solidification when one works on the basis of natural fatty alcohols.

The tube reactor is preferably operated in the transition area between laminar and turbulent flow or in the turbulent area, such that a homogeneous and uniform reaction profile is created. Practically, as mentioned before, this turbulence is created by applying flow rates which are so high that a Reynolds' number of at least 10⁵ is maintained.

In order to manufacture at an industrial level, one or several tube reactors are preferably applied, having a diameter of 2.5 cm to 2 m and a total length of 10 to 300 m.

Since the product increases in mass and density during the reaction, one or several tube reactors are preferably used whose flow diameters systematically increase according to the direction of flow, and/or several tube reactors are used whose successive flow diameters increase.

Practically, the total reactor volume in the tube reactor or tube reactors is situated between 1 m³ and 30 m³.

In order to initially advance the reaction, a pre-heating can be applied until a temperature in the order of magnitude of 100 to 150°C is reached.

Especially in the case of a continuous process, several tube reactors are preferably placed in series.

In this case, intercoolers can be provided between the tube reactors, so as to prevent the product temperature from rising too much, and so as to cool the product down again to a temperature which is sufficiently low in order to allow for another adiabatic reaction in a following tube reactor.

The entire amount of ethylene oxide and/or propylene oxide does not necessarily have to be supplied at the entry of the tube reactor; a part thereof can also be supplied somewhat further at one or several other places. Practically, this extra supply is preferably systematically provided for between two or several of such tube reactors placed in series, whereby an almost complete ethylene and/or propylene oxide conversion per tube reactor is always aimed at.

The invention also concerns a device for realising the above-mentioned method, characterised in that it shows the combinations of features of annexed claim 20, or of annexed claim 21.

This device can be further distinguished from all existing embodiments in that it has a simple construction, which results in serious savings on the capital outlay for a new production unit. In an ideal manner, the device is integrated downstream in an ethylene or propylene oxide factory.

In order to better explain the characteristics of the invention, the following preferred embodiments are described as an example only without being limitative in any way, with reference to the accompanying drawings, in which:
figure 1 schematically represents a device according to the invention;
figure 2 represents a variant of the device according to the invention.

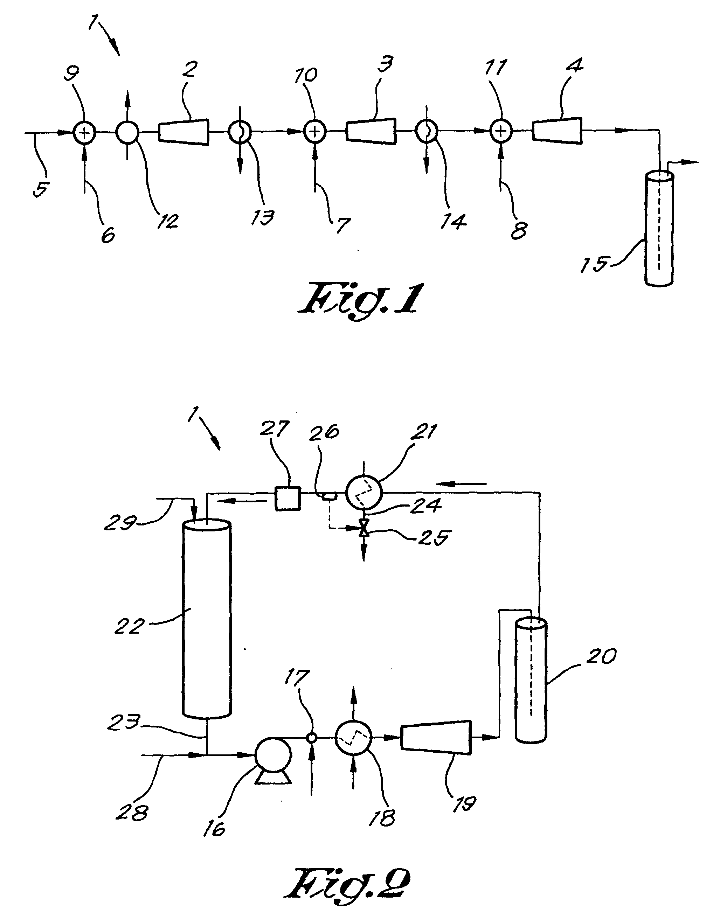

Figure 1 represents a device 1 according to the invention, whereby several tube reactors, in this case three as indicated by the references 2, 3 and 4, are erected in series, one after the other.

The device 1 contains a feeding device 5 for alcohol, as well as a feeding device 6 for ethylene oxide and/or propylene oxide.

Further, a number of extra supply points 7 and 8 for ethylene oxide and/or propylene oxide are provided.

In order to obtain a good mixture, the necessary mixers 9, 10 and 11 are integrated in the device 1. These mixers 9, 10 and 11 can be of any nature whatsoever, and they may either or not contain agitators. According to a simple embodiment, such a mixer may consist of a through pipe for the alcohol in which one or several mouthpieces open via which the ethylene oxide and/or propylene oxide are supplied.

Finally, the device 1 on the one hand contains a pre-heating device 12 to initially heat the reagents, and a number of coolers on the other hand, 13 and 14 respectively, in particular intercoolers with which intercoolings are realised before new reagents are supplied.

The working of the device 1 and the method applied are as follows:

Via the feeding devices 5 and 6, the reagents concerned are provided in the device 1.

Before the mixed reagents are supplied to the first tube reactor 2, the mixture is pre-heated in the pre-heating device 12, at a temperature in the order of magnitude of for example 130°C.

An adiabatic reaction is realised in the tube reactor 2.

In order to prevent the product temperature from rising too much, there is an intercooling in a cooler 13, for example until the temperature drops again to some 130°C.

Next, the preceding step is repeated. It consists in admixing ethylene oxide and/or propylene oxide via the supply 7, in making the whole react in the tube reactor 3, and in letting the product obtained from this reactor cool down in a cooler 14.

Eventually, this is repeated once more with the help of the mixer 11 and the tube reactor 4. In the end, the product can cool down by itself and a forced cooling is not really necessary.

It should be noted that the tube reactors 2, 3 and 4 are represented only very schematically. In reality, they consist of rather long, tubular elements, in particular having sizes in the order of magnitude as mentioned in the introduction. The tubular design of the reactors makes it possible to guarantee a fast, unidirectional turbulent flow.

It is clear that the reagents are supplied under sufficient pressure in order to obtain that the product moves at an appropriate speed through the device 1. In order to guarantee a homogenous liquid phase reaction, it is necessary to keep the pressure of the reaction medium somewhat above the vapour pressure of the reaction mixture.

It is clear that the number of tube reactors is not limited to a certain number. Practically, in order to be able to carry out the reaction from beginning to end, three to five tube reactors 2-3-4 are preferably applied.

The device 1 of figure 1 allows for a continuous production. This continuous production does not exclude, however, that the end product can be kept in a finishing reactor 15 for a while in order to completely finish the reaction of the product. Said finishing reactor 15 can simultaneously act as a receiving tank.

Although the continuous reaction is the most appropriate, it is not excluded to carry out the reaction as a semi-batch process, for example in the case of smaller production quantities. An example of this is represented in figure 2.

The device 1 in this case has a cyclic circulation and contains a circulating pump 16; a supply point 17, either or not with a special mixer, for the ethylene oxide and/or propylene oxide; a pre-heating device 18; at least one tube reactor 19; a finishing reactor 20; a cooler 21; and a surge tank 22 which is in turn connected to the tube reactor 19 via a canalisation 23. In the pipe 24 for the cooling medium of the cooler 21 is provided a control valve 25 which is coupled to a temperature sensor 26 situated at the outlet of the cooler 21. The pressure of the reactor is controlled by means of a valve 27.

The working of the device 1 of figure 2 is mainly as follows:

Via a supply 28, a basic product such as detergent alcohol or glycol is put in the device 1. By means of the circulating pump 16, the product is pumped round. In the pre-heating device 18, a pre-heating is carried out up to about 130°C.

As soon as this temperature has been reached, ethylene oxide and/or propylene oxide is supplied via the supply point 17. The flow rate of the ethylene oxide and/or propylene oxide hereby depends on the recirculation flow rate, on the required length of time during which it has to stay in the reactor in order to guarantee a complete ethylene and/or propylene oxide conversion, and on the produced reaction heat, in particular the admitted adiabatic rise in temperature.

The total amount of ethylene oxide or propylene oxide that has to be supplied is determined by the starting product and the required end product.

As mentioned above, the main reaction is carried out in the tube reactor 19.

In the finishing reactor 20, the reaction of the last remainders of ethylene oxide and/or propylene oxide can be completely finished. The outlet pressure on the outlet of the finishing reactor is set at an operating pressure which is higher than the vapour pressure of the mixture, preferably higher than 10 bar.

Subsequently, the reaction product is cooled with water in the cooler 21.

The accretion of the reaction product, resulting from adding the ethylene oxide and/or propylene oxide, is collected in the surge tank 22. The product simply flows from the cooler 21 in the surge tank 22 via the top and falls down according to what is called the splash filling principle. In this manner is obtained that the principle of the slug flow is maintained.

Via a feeding device 29, a nitrogen control can be provided for so as to remain outside the explosive range of the gas mixture and so as to be able to guarantee the colour stability of the ethoxylate or propoxylate or such.

In general, the outlet temperature at the finishing reactor 15 is limited to 200°C.

In the case of a device 1 according to figure 2, the circulating batch material will be entirely cooled as soon as the required number of ethylene oxide adducts have been provided on the basic product.

In the end, a finishing step can also be provided for, whereby for example a neutralisation means is put in the device 1 via the supply 28.

It should be noted that both in the continuous process of figure 1 and in the process of figure 2, also other products can be added to the basic product, such as a catalyst, for example KOH, and possibly a reduction agent, for example NaBH₄.

It is not excluded to place two or several tube reactors after one another in the semi-batch process as well.

Although the detailed embodiment refers to an ethoxylation and/or a propoxylation, it is clear that the same applies to similar reactions, such as butoxylation or such.

## Claims

1. Method for manufacturing products such as surfactants and polyethylene glycols by means of an alkoxylation reaction, **characterized in that** it at least consists of putting the reagents together in the liquid phase, and of subsequently leading the reaction medium in which the reaction takes place at least for the greater part through one or several tube reactors (2-3-4; 19), whereby in the tube reactor, the above-mentioned reaction is carried out in an adiabatic manner, and whereby a turbulence is created in the tube reactor (2-3-4; 19) by maintaining a Reynolds' number of at least 10⁵.

2. Method according to claim 1, **characterised in that** it is used for the production of alcohol alkoxylates, whereby an ethoxylation and/or propoxylation takes place by making react alcohol and/or another basic product on the one hand with ethylene oxide and/or propylene oxide on the other hand.

3. Method according to claim 1, **characterised in that** it is used for the production of alcohol alkoxylates, whereby a butoxylation takes place.

4. Method according to claim 1, **characterised in that** it is used for the production of alcohol alcoxylates of one or several of the following types:
a. primary alcohol ethoxylates 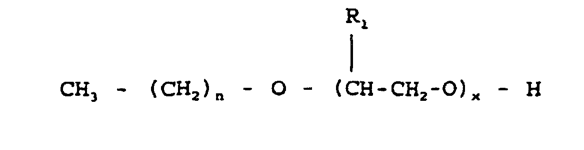
b. secondary alcohol ethoxylates 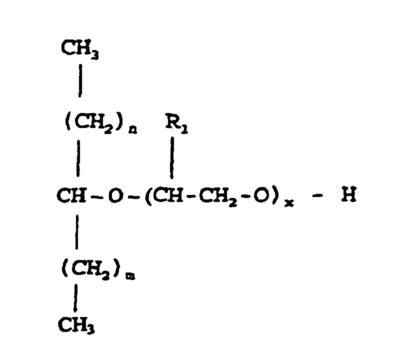
c. tertiary alcohol ethoxylates 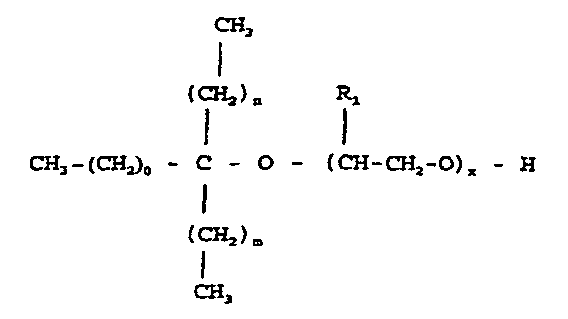 Whereby:R₁ consists of H, CH₃ or C₂H₅
x = 0-50
m,n,o = 0-20

5. Method according to claim 1, **characterised in that** it is used for the production of polyalkylene glycols, in particular of the following type: 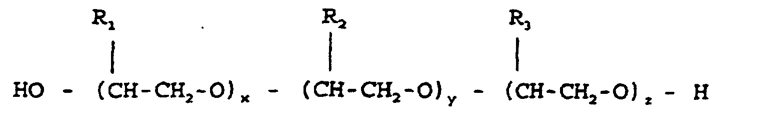 Whereby: R₁,R₂,R₃ consist of H, CH₃ or C₂H₅
x,y,z = 0-50

6. Method according to any of the preceding claims, **characterised in that** it is realised as a continuous process.

7. Method according to any of claims 1 to 5, **characterised in that** it is realised in the form of a semi-batch process.

8. Method according to any of the preceding claims, **characterised in that** a tube reactor (2-3-4;19) is used having a diameter of 2.5 to 2 m.

9. Method according to any of the preceding claims, **characterised in that** a tube reactor (2-3-4;19) or several reactors (2-3-4;19) are used having a total length of 10 to 300 m.

10. Method according to any of the preceding claims, **characterised in that** one or several tube reactors (2-3-4;19) are used whose flow diameters systematically increase depending on the direction of flow, and/or **in that** several tube reactors are used in series whose successive flow diameters increase.

11. Method according to any of the preceding claims, **characterised in that** the total reactor volume of the tube reactors (2-3-4;19) is situated between 1 m³ and 30 m³.

12. Method according to any of the preceding claims, **characterised in that**, after the reagents have been put together, a pre-heating is provided for before the whole is supplied to the tube reactor (2-3-4;19).

13. Method according to claim 12, **characterised in that** a pre-heating is provided for up to a temperature in the order of magnitude of 100 to 150°C.

14. Method according to any of the preceding claims, **characterised in that** the obtained product is cooled.

15. Method according to any of the preceding claims, **characterised in that** use is made of two or several tube reactors (2-3-4) connected in series.

16. Method according to claim 15, **characterised in that** an intercooling is carried out between two or several of the tube reactors (2-3-4;19).

17. Method according to any of the preceding claims, **characterised in that**, after the reaction has started, additional reagent is supplied, in particular ethylene oxide and/or propylene oxide.

18. Method according to any of claims 15 or 16 and according to claim 17, **characterised in that** the additional supply is provided for in between the tube reactors (2-3-4) connected in series.

19. Method according to any of the preceding claims, **characterized in that**, as a finishing operation, the obtained product is put in a finishing reactor (15-20).

20. Device for realising the method according to any of claims 1 to 5, **characterized in that** it at least consists of at least one feeding device (5-6) to supply reagents which together by means of an alkoxylation reaction can react to a surfactant or a polyethylene glycol; at least one mixer (9-10-11) mixing said reagents in the liquid phase; a pre-heating device (12) following the first mixer (9); at least two tube reactors (2-3-4) which are erected in series in which the mixed reagents can react, said tube reactors (2-3-4) being provided of means which permit that the reagents react in adiabatic conditions and under turbulences which are **characterized by** a Reynolds' number of at least 10⁵; at least one additional supply (7-8) between two or several of the tube reactors (2-3-4) via which ethylene oxide and/or propylene oxide can be supplied; and one or several intercoolers (13-14).

21. Device for realising the method according to any of claims 1 to 5, **characterized in that** it at least consists of at least one feeding device (5-6) to supply reagents which together by means of an alkoxylation reaction can react to a surfactant or a polyethylene glycol; at least one mixer mixing said reagents in the liquid phase; at least one tube reactor (19) in which the mixed reagents can react, said tube reactor (19) being configured such that the reagents react in adiabatic conditions and under turbulences which are **characterized by** a Reynolds' number of at least 10⁵; and **characterised in that** it contains a cyclic circulation whereby the end of the tube reactor (19) is connected either directly or indirectly to a surge tank (22) which is in turn connected to the inlet of the tube reactor (19) via canalisation; and where the outlet of the tube reactor (19) is connected to a finishing reactor (20) in the shape of a recipient, and **in that** a cooler (21) is provided between the finishing reactor (20) and the surge tank (22).

22. Device according to any of claims 20 to 21, **characterised in that** the tube reactor (2-3-4;19) or reactors have dimensions and/or a shape which correspond to the characteristics described in claims 8 and/or 9.

23. Device according to any of claims 20 to 22, **characterised in that** it is integrated downstream in an ethylene and/or propylene oxide factory.

## Patentansprüche

1. Verfahren zur Herstellung von Produkten wie z.B. Tensiden und Polyethylenglykolen durch eine Alkoxylationsreaktion, **dadurch gekennzeichnet, dass** es mindestes darin besteht, die Reagenzien in der Flüssigphase zusammenzubringen und anschließend das Reaktionsmedium, in dem die Reaktion zumindest zum größeren Teil stattfindet, durch einen oder mehrere Röhrenreaktoren (2-3-4; 19) zu führen, wobei in dem Röhrenreaktor die oben beschriebene Reaktion in adiabatischer Weise ausgeführt wird und wobei ein Wirbel in dem Röhrenreaktor (2-3-4; 19) erzeugt wird, indem man eine Reynoldsche Zahl von mindestens 10⁵ aufrechterhält.

2. Verfahren nach Anspruch 1, **dadurch gekennzeichnet, dass** es zur Herstellung von Alkoholalkoxylaten benutzt wird, wobei eine Ethoxylation und/oder Propoxylation stattfindet, indem man Alkohol und/oder ein anderes Basisprodukt mit Ethylenoxid einerseits und/oder mit Propylenoxid andererseits reagieren lässt.

3. Verfahren nach Anspruch 1, **dadurch gekennzeichnet, dass** es zur Herstellung von Alkoholalkoxylaten benutzt wird, wobei eine Butoxylation stattfindet.

4. Verfahren nach Anspruch 1, **dadurch gekennzeichnet, dass** es zur Herstellung von Alkoholalkoxylaten von einer oder mehreren der folgenden Arten benutzt wird:
a. primäre Alkoholethoxylate 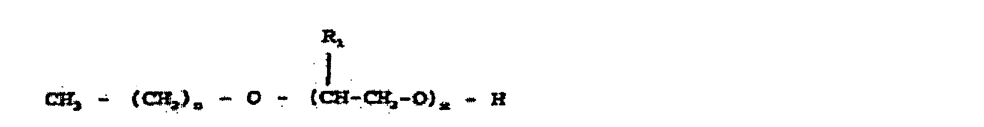
b. sekundäre Alkoholethoxylate 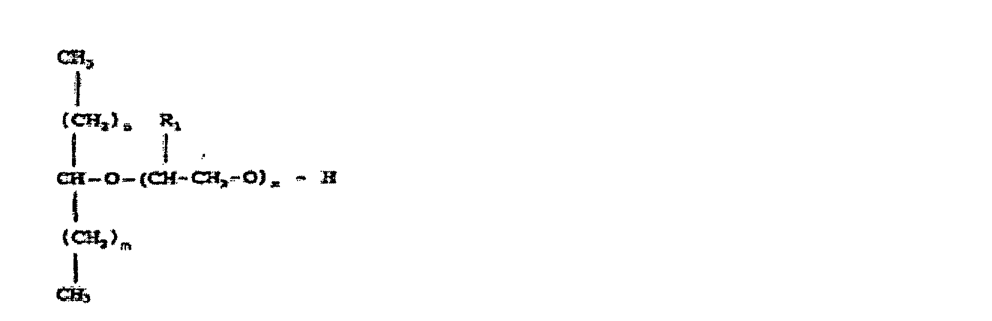
c. tertiäre Alkoholethoxylate 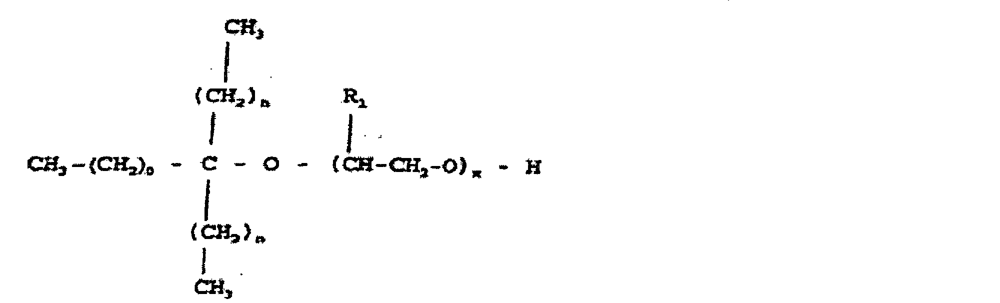 Wobei:R₁ aus H, CH₃ oder C₂H₅ besteht
x = 0-50
m,n,o = 0-20

5. Verfahren nach Anspruch 1, **dadurch gekennzeichnet, dass** es zur Herstellung von Polyalkylenglykolen benutzt wird, insbesondere der folgenden Art: 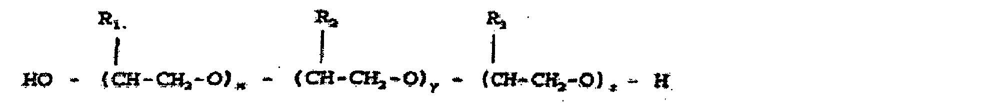 Wobei:R₁, R₂, R₃ aus H, CH₃ oder C₂H₅ besteht
x, y, z = 0-50

6. Verfahren nach einem der vorhergehenden Ansprüche, **dadurch gekennzeichnet, dass** es als kontinuierlicher Prozess ausgeführt wird.

7. Verfahren nach einem der Ansprüche 1 bis 5, **dadurch gekennzeichnet, dass** es in der Form eines Halbbatchprozesses ausgeführt wird.

8. Verfahren nach einem der vorhergehenden Ansprüche, **dadurch gekennzeichnet, dass** ein Röhrenreaktor (2-3-4; 19) verwendet wird, der einen Durchmesser von 2,5 bis 2 m hat.

9. Verfahren nach einem der vorhergehenden Ansprüche, **dadurch gekennzeichnet, dass** ein Röhrenreaktor (2-3-4; 19) oder mehrere Röhrenreaktoren (2-3-4; 19) verwendet werden, die eine Gesamtlänge von 10 bis 300 m haben.

10. Verfahren nach einem der vorhergehenden Ansprüche, **dadurch gekennzeichnet, dass** ein oder mehrere Röhrenreaktoren (2-3-4; 19) verwendet werden, deren Fließdurchmesser in Abhängigkeit von der Fließrichtung systematisch zunehmen und/oder dass mehrere Röhrenreaktoren in Reihe verwendet werden, deren aufeinander folgende Fließdurchmesser zunehmen.

11. Verfahren nach einem der vorhergehenden Ansprüche, **dadurch gekennzeichnet, dass** das gesamte Reaktorvolumen der Röhrenreaktoren (2-3-4; 19) zwischen 1 m³ und 30 m³ beträgt.

12. Verfahren nach einem der vorhergehenden Ansprüche, **dadurch gekennzeichnet, dass** nach dem Zusammenbringen der Reagenzien für eine Vorerwärmung gesorgt ist, bevor das Ganze dem Röhrenreaktor (2-3-4; 19) zugeführt wird.

13. Verfahren nach Anspruch 12, **dadurch gekennzeichnet, dass** für eine Vorerwärmung auf eine Temperatur im Bereich von 100 bis 150°C gesorgt ist.

14. Verfahren nach einem der vorhergehenden Ansprüche, **dadurch gekennzeichnet, dass** das erhaltene Produkt gekühlt wird.

15. Verfahren nach einem der vorhergehenden Ansprüche, **dadurch gekennzeichnet, dass** zwei oder mehrere Röhrenreaktoren (2-3-4), die in Reihe angeordnet sind, verwendet werden.

16. Verfahren nach Anspruch 15, **dadurch gekennzeichnet, dass** eine Zwischenkühlung zwischen zwei oder mehreren der Röhrenreaktoren (2-3-4; 19) ausgeführt wird.

17. Verfahren nach einem der vorhergehenden Ansprüche, **dadurch gekennzeichnet, dass**, nachdem die Reaktion begonnen hat, ein zusätzliches Reagens zugeführt wird, insbesondere Ethylenoxid und/oder Propylenoxid.

18. Verfahren nach einem der Ansprüche 15 oder 16 und nach Anspruch 17, **dadurch gekennzeichnet, dass** die zusätzliche Zufuhr zwischen den in Reihe angeordneten Röhrenreaktoren (2-3-4) bereit gestellt ist.

19. Verfahren nach einem der vorhergehenden Ansprüche, **dadurch gekennzeichnet, dass** als Nacharbeit das erhaltene Produkt in einen Nachreaktor (15-20) gegeben wird.

20. Einrichtung zum Ausführen des Verfahrens nach einem der Ansprüche 1 bis 5, **dadurch gekennzeichnet, dass** sie aus mindestens einer Zuführeinrichtung (5-6) zum Zuführen von Reagenzien, die zusammen durch eine Alkoxylationsreaktion auf ein Tensid oder ein Polyethylenglykol reagieren können; mindestens einem Mischer (9-10-11) zum Mischen der Reagenzien in der Flüssigphase; einer Vorwärmeinrichtung (12) im Anschluss an den ersten Mischer (9); mindestens zwei Röhrenreaktoren (2-3-4), die in Reihe angeordnet sind, in denen die gemischten Reagenzien reagieren können, wobei die Röhrenreaktoren (2-3-4) mit Mitteln versehen sind, die es ermöglichen, dass die Reagenzien in adiabatischen Bedingungen und unter Wirbeln reagieren, die durch eine Reynoldsche Zahl von mindestens 10⁵ gekennzeichnet sind; mindestens einer zusätzlichen Zufuhr (7-8) zwischen zwei oder mehreren der Röhrenreaktoren (2-3-4), über die Ethylenoxid und/oder Propylenoxid zugeführt werden kann; und einem oder mehreren Zwischenkühlern (13-14) besteht.

21. Einrichtung zum Ausführen des Verfahrens nach einem der Ansprüche 1 bis 5, **dadurch gekennzeichnet, dass** sie mindestens aus mindestens einer Zuführvorrichtung (5-6) zum Zuführen von Reagenzien, die zusammen durch eine Alkoxylationsreaktion auf ein Tensid oder ein Polyethylenglykol; mindestens einem Mischer zum Mischen der Reagenzien in der Flüssigphase; mindestens einem Röhrenreaktor (19), in dem die gemischten Reagenzien reagieren können, wobei der Röhrenreaktor (19) so konfiguriert ist, dass die Reagenzien in adiabatischen Bedingungen und unter Wirbeln reagieren, die durch eine Reynoldsche Zahl von mindestens 10⁵ gekennzeichnet sind, besteht; und **dadurch gekennzeichnet ist, dass** sie einen zyklischen Umlauf enthält, wobei das Ende des Röhrenreaktors (19) entweder direkt oder indirekt mit einem Auffangbehälter (22) verbunden ist, das wiederum über eine Rohrleitung mit dem Einlass des Röhrenreaktors (19) verbunden ist; und wobei der Auslass des Röhrenreaktors (19) mit einem Nachreaktor (20) in der Form eines Ballons verbunden ist und dass ein Kühler (21) zwischen dem Nachreaktor (20 und dem Auffangbehälter (22) bereit gestellt ist.

22. Einrichtung nach einem der Ansprüche 20 bis 21, **dadurch gekennzeichnet, dass** der Röhrenreaktor (2-3-4; 19) oder die Röhrenreaktoren Abmessungen und/oder eine Form haben, die den in Ansprüchen 8 und/oder 9 beschriebenen Merkmalen entsprechen.

23. Einrichtung nach einem der Ansprüche 20 bis 22, **dadurch gekennzeichnet, dass** sie stromabwärts in einer Ethylen- und/oder Propylenoxidfabrik integriert ist.

## Revendications

1. Procédé pour fabriquer des produits tels que des agents tensioactifs et des polyéthylèneglycols au moyen d'une réaction d'alcoxylation, **caractérisé en ce qu'**il consiste au moins à réunir les réactifs en phase liquide et à guider ensuite le milieu réactionnel dans lequel a lieu la réaction au moins pour sa majeure partie à travers un ou plusieurs réacteurs tubulaires (2-3-4 ; 19), , la réaction mentionnée ci-dessus étant mise en oeuvre dans le réacteur tubulaire d'une manière adiabatique et une turbulence étant créée dans le réacteur tubulaire (2-3-4 ; 19) en maintenant un nombre de Reynolds d'au moins 10⁵.

2. Procédé selon la revendication 1, **caractérisé en ce qu'**on l'utilise pour la production d'alcoxylates d'alcools, en mettant en oeuvre une éthoxylation et/ou une propoxylation en faisant réagir un alcool et/ou un autre produit de base d'une part avec de l'oxyde d'éthylène et/ou de l'oxyde de propylène d'autre part.

3. Procédé selon la revendication 1, **caractérisé en ce qu'**on l'utilise pour la production d'alcoxylates d'alcools, en mettant en oeuvre une butoxylation.

4. Procédé selon la revendication 1, **caractérisé en ce qu'**on l'utilise pour la production d'alcoxylates d'alcools d'un ou de plusieurs types choisis parmi ceux repris ci-après :
a. des éthoxylates d'alcools primaires 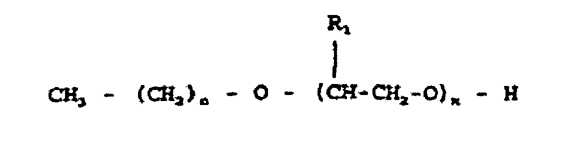
b. des éthoxylates d'alcools secondaires 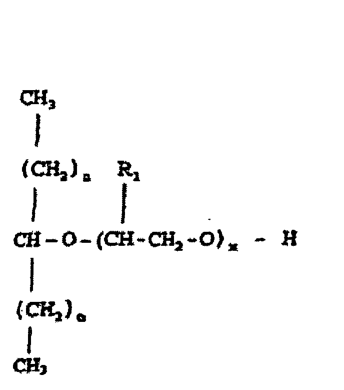
c. des éthoxylates d'alcools tertiaires 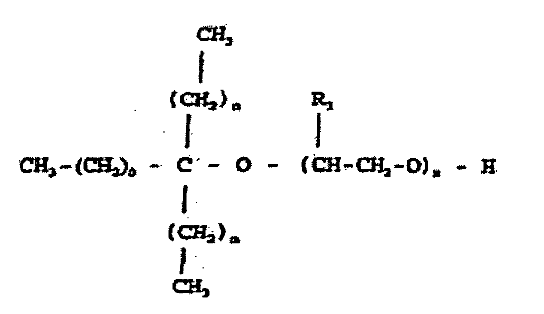
dans lesquels
R₁ représente un atome d'hydrogène, un groupe CH₃ ou un groupe C₂H₅ ;
x = 0 - 50
m, n, o = 0 - 20.

5. Procédé selon la revendication 1, **caractérisé en ce qu'**on l'utilise pour la production de polyalkylèneglycols, en particulier ceux du type ci-après : 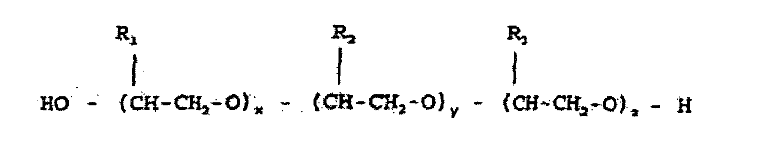 dans lequel
R₁, R₂, R₃ représentent un atome d'hydrogène, un groupe CH₃ ou un groupe C₂H₅ ;
x = 0 - 50.

6. Procédé selon l'une quelconque des revendications précédentes, **caractérisé en ce qu'**on le met en oeuvre sous la forme d'un procédé en continu.

7. Procédé selon l'une quelconque des revendications 1 à 5, **caractérisé en ce qu'**on le met en oeuvre sous la forme d'un procédé en semi-continu.

8. Procédé selon l'une quelconque des revendications précédentes, **caractérisé en ce qu'**on utilise un réacteur tubulaire (2-3-4 ; 19) possédant un diamètre de 2,5 à 2 m.

9. Procédé selon l'une quelconque des revendications précédentes, **caractérisé en ce qu'**on utilise un réacteur tubulaire (2-3-4 ; 19) ou plusieurs réacteurs tubulaires (2-3-4 ; 19) dont la longueur totale s'élève de 10 à 300 m.

10. Procédé selon l'une quelconque des revendications précédentes, **caractérisé en ce qu'**on utilise un ou plusieurs réacteurs tubulaires (2-3-4 ; 19) dont les diamètres d'écoulement augmentent de manière systématique en fonction de la direction d'écoulement et/ou **en ce qu'**on utilise plusieurs réacteurs tubulaires montés en série dont les diamètres d'écoulement successifs augmentent.

11. Procédé selon l'une quelconque des revendications précédentes, **caractérisé en ce que** le volume total des réacteurs tubulaires (2-3-4 ; 19) se situe entre 1 m³ et 30 m³.

12. Procédé selon l'une quelconque des revendications précédentes, **caractérisé en ce que**, après avoir réuni les réactifs, on procure un préchauffage avant d'acheminer le tout aux réacteurs tubulaires (2-3-4 ; 19).

13. Procédé selon la revendication 12, **caractérisé en ce qu'**on procure un préchauffage jusqu'à une température qui se situe dans l'ordre de grandeur de 100 à 150 °C.

14. Procédé selon l'une quelconque des revendications précédentes, **caractérisé en ce que** le produit obtenu est refroidi.

15. Procédé selon l'une quelconque des revendications précédentes, **caractérisé en ce qu'**on fait usage de deux réacteurs tubulaires (2-3-4) ou plus montés en série.

16. Procédé selon la revendication 15, **caractérisé en ce qu'**on met en oeuvre un refroidissement intermédiaire entre deux ou plusieurs réacteurs parmi lesdits réacteurs tubulaires (2-3-4 ; 19) montés en série.

17. Procédé selon l'une quelconque des revendications précédentes, **caractérisé en ce que**, après le démarrage de la réaction, on alimente un réactif supplémentaire, en particulier de l'oxyde d'éthylène et/ou de l'oxyde de propylène.

18. Procédé selon l'une quelconque des revendications 15 ou 16 et selon la revendication 17, **caractérisé en ce que** l'alimentation supplémentaire a lieu entre les réacteurs tubulaires (2-3-4) montés en série.

19. Procédé selon l'une quelconque des revendications précédentes, **caractérisé en ce que**, à titre d'opération de finition, on place le produit obtenu dans un réacteur de finition (15-20).

20. Dispositif de mise en oeuvre du procédé selon l'une quelconque des revendications 1 à 5, **caractérisé en ce qu'**il est au moins constitué par au moins un dispositif de charge (5-6) pour alimenter des réactifs qui, via une réaction d'alcoxylation, sont en mesure de réagir les uns avec les autres pour obtenir un agent tensioactif ou un polyéthylène glycol ; au moins un mélangeur (9-10-11) pour le mélange desdits réactifs en phase liquide ; un dispositif de préchauffage (12) à la suite du premier mélangeur (9) ; au moins deux réacteurs tubulaires (2-3-4) qui sont montés en série, dans lesquels les réactifs mélangés peuvent réagir, lesdits réacteurs tubulaires (2-3-4) étant munis de moyens qui permettent aux réactifs de réagir dans des conditions adiabatiques et en présence de turbulences qui sont **caractérisées par** un nombre de Reynolds d'au moins 10⁵ ; au moins une alimentation supplémentaire (7-8) entre deux ou plusieurs réacteurs parmi les réacteurs tubulaires (2-3-4), par laquelle on peut alimenter de l'oxyde éthylène et/ou de l'oxyde de propylène ; et un ou plusieurs dispositifs de refroidissement intermédiaires (13-14).

21. Dispositif de mise en oeuvre du procédé selon l'une quelconque des revendications 1 à 5, **caractérisé en ce qu'**il est au moins constitué par au moins un dispositif de charge (5-6) pour alimenter des réactifs qui, via une réaction d'alcoxylation, sont en mesure de réagir les uns avec les autres pour obtenir un agent tensioactif ou un polyéthylène glycol ; au moins un réacteur tubulaire (19) dans lequel les réactifs mélangés peuvent réagir, ledit réacteur tubulaire (19) étant configuré de telle sorte que les réactifs réagissent dans des conditions adiabatiques et en présence de turbulences qui sont **caractérisées par** un nombre de Reynolds d'au moins 10⁵ ; et **caractérisé en ce qu'**il contient une circulation cyclique par laquelle l'extrémité du réacteur tubulaire (19) est raccordée, soit directement, soit indirectement à un réservoir d'égalisation de pression (22) qui est raccordé à son tour à l'entrée du réacteur tubulaire (19) via une canalisation ; et dans lequel la sortie du réacteur tubulaire (19) est raccordée à un réacteur de finition (20) en forme de récipient, et **en ce qu'**on prévoit un dispositif de refroidissement (21) entre le réacteur de finition (20) et le réservoir d'égalisation de pression (22).

22. Dispositif selon l'une quelconque des revendications 20 à 21, **caractérisé en ce que** le ou les réacteurs tubulaires (2-3-4 ; 19) possède des dimensions et/ou une configuration qui correspondent aux caractéristiques décrites dans les revendications 8 et/ou 9.

23. Dispositif selon l'une quelconque des revendications 20 à 22, **caractérisé en ce qu'**il est intégré en aval dans une usine de fabrication d'oxyde d'éthylène et/ou d'oxyde de propylène.
